(19) Europäisches Patentamt
European Patent Office
Office européen des brevets

(11) EP 4 736 936 A1

(12) EUROPEAN PATENT APPLICATION

(43) Date of publication:
06.05.2026 Bulletin 2026/19

(21) Application number: 24210873.6

(22) Date of filing: 05.11.2024

(51) International Patent Classification (IPC):
A61N 1/365 (2006.01)        A61B 5/349 (2021.01)
A61N 1/39 (2006.01)

(52) Cooperative Patent Classification (CPC):
A61N 1/365; A61B 5/349; A61N 1/39622

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(71) Applicant: BIOTRONIK SE & Co. KG
12359 Berlin (DE)

(72) Inventors:
• Weiss, Ingo
  75173 Pforzheim (DE)
• Ciesla, Catharina-Sophie
  12167 Berlin (DE)

(74) Representative: Biotronik Corporate Services SE
Corporate Intellectual Property
Sieversufer 9
12359 Berlin (DE)

(54) IMPLANTABLE MEDICAL SYSTEM AND METHOD FOR OPERATING

(57) The present invention relates to an implantable medical system for stimulating a patient's heart, comprising a memory unit (270), a processor (28), a stimulation unit (290) configured to stimulate the heart, and a detection unit (260) configured to detect a cardiac electric signal (250). The memory unit (270) stores a parameter allocation model which comprises a plurality of key sets (310, 420), each of which comprises a potential time of occurrence of at least one first event (110) in the cardiac electric signal (250) and each of which is mapped to at least one potential time of occurrence of a second event (130) in the cardiac electric signal (250) representing a repolarization of the myocardium. The memory unit (270) further stores a computer-readable read-out program that causes the processor (28) to perform the following steps when executed on the processor (28):
- receiving a cardiac electric signal (250) comprising a plurality of events (110),
- for at least one first event (110) in the received cardiac electric signal (250), determining an actual time of occurrence,
- finding the key set in the list of key sets of the parameter allocation model that is closest to the actual time of occurrence, and
- retrieving an estimation time from the parameter allocation model, which represents an estimation of the time of occurrence of the second event (130), based on the at least one potential time of occurrence of the at least one second event (130) to which the closest key set is mapped.

EP 4 736 936 A1

FIG. 4A

**Description**

The present invention relates to an implantable medical system according to the preamble of claims 1 and 12 and to a method of operating an implantable medical system according to the preamble of claim 15.

The implantable medical system may comprise a non-transvenous implantable cardioverter-defibrillator device generally designed for implantation external to a patient's heart. A non-transvenous implantable cardioverter-defibrillator device, in short non-transvenous ICD, comprises a stimulation unit and a processor, and at least one lead comprising a shock electrode for emitting an electrical shock pulse externally to a patient's heart. The lead is connected to the stimulation unit. The stimulation unit forms part of a generator device that may, for example, be implanted subcutaneously in a patient. The lead, in a connected state, extends from the generator device, the lead being implanted such that it fully rests outside of the patient's heart. The lead may for example extend from the generator device towards a location in the region of the patient's sternum, the shock electrode hence being placed outside of the patient's heart for emitting an electrical shock pulse at a location external to the patient's heart.

The term "non-transvenous" in this respect, in particular, shall express that the lead of the non-transvenous implantable cardioverter-defibrillator device does not extend transvenously into the heart, but fully rests outside of the patient's heart.

The implantable medical system comprising the non-transvenous implantable cardioverter-defibrillator device, in particular, is designed for emitting electrical shocks in case life-threatening arrhythmias of a patient's heart are detected. By means of an electrical shock, a defibrillation shall be achieved in order to reset the cardiac rhythm back to a normal state.

In some instances, T wave shocks are applied with a fixed, manually predetermined delay of the shock with respect to the previous QRS complex in the electrocardiogram (Barold, H. S., & Wharton, J. M. (1997)). Ventricular fibrillation resulting from synchronized internal atrial defibrillation in a patient with ventricular preexcitation. Journal of Cardiovascular Electrophysiology, 8(4), 436-440). However, this approach does not consider possible variations in the cardiac rhythm of the patient.

When operating the implantable medical system, it is helpful to estimate a time of occurrence of the next T wave of the cardiac rhythm of the patient. Typically, shock pulses for achieving a cardioversion are applied outside the T wave. If the cardioversion would be performed during the T wave, there is a high risk of inducing ventricular fibrillation by the cardioversion shock pulses.

In some instances, such ventricular fibrillation of the patient's heart is desired. For this purpose, sequences of pulse bursts are emitted by the shock electrode. As such pulse bursts may be emitted over a duration of several seconds and may be delivered with substantial energy, such pulse bursts may cause a significant stress on a patient and its muscular system, causing potentially postoperative pain to a patient. R-wave synchronized inducing of ventricular fibrillation has been described by Wylie Jr. et al. (Wylie Jr, J. V., Essebag, V., Reynolds, M. R., & Josephson, M. E. (2009). Inducibility of Atrial Fibrillation with a Synchronized External Low Energy Shock Post-Pulmonary Vein Isolation Predicts Recurrent Atrial Fibrillation. Journal of Cardiovascular Electrophysiology, 20(1), 29-36). However, an exact knowledge on the time of occurrence of the T wave increases the efficacy of such pulse bursts.

In some instances, an electric (or galvanically coupled) communication between different implantable or implanted medical devices is desired. The risk of ventricular fibrillation is significantly reduced if any electric communication pulses are emitted by such devices only outside the T waves of the patient's cardiac rhythm.

Thus, there is a general need of an estimation of the time of occurrence of the T wave in the patient's cardiac rhythm for these different applications of emitting electric pulses during or outside the T wave.

It is an object of the present invention to provide an implantable medical system for stimulating a patient's heart, comprising a memory unit, a processor, a stimulation unit configured to stimulate the heart, and a detection unit configured to detect a cardiac electric signal and a method for operating such system, which allow for a more exact estimation of the occurrence of the T wave in a patient's cardiac rhythm.

According to a first aspect of the invention, this object is achieved with an implantable medical system wherein the memory unit stores a parameter allocation model which comprises a plurality of key sets, each of which comprises a potential time of occurrence of at least one first event in the cardiac electric signal and each of which is mapped to at least one potential time of occurrence of a second event in the cardiac electric signal representing a repolarization of the myocardium, wherein the memory unit further stores a computer-readable read-out program that causes the processor to perform the following steps when executed on the processor:

- receiving a cardiac electric signal comprising a plurality of events,
- for at least one first event in the received cardiac electric signal, determining an actual time of occurrence,
- finding the key set in the list of key sets of the parameter allocation model that is closest to the actual time of occurrence, and
- retrieving an estimation time from the parameter allocation model, which represents an estimation of the time of occurrence of the second event, based on the at least one potential time of occurrence of the at least one second event to which the closest key set is mapped.

By using the parameter allocation model, the implantable medical system is capable of estimating the occurrence of, for example the T wave as a form of a second event representing the repolarization of the myocardium, more exactly. Specifically, a preciseness of estimation of the time of occurrence of the T wave may be better than +/- 100 ms, +/- 50 ms or even better than +/- 20 ms. Hereby, the estimation is based on the at least one potential time of occurences of the at least one second event. This may be a time at which the at least one second event has occurred in the past. Thus, the parameter allocation model may be understood to serve as a reference for times of occurrence of the second event in the past. These may be used as potential times of occurrence of the at least one second event in the future for improving the estimation of an actual time of occurrence of the at least one second event.

Thus, the implantable medical system may rely on times of occurrence of the second event in the past (or at least values derived from these) to retrieve the estimation time such that computing-intensive calculations are avoided. In doing so, a battery capacity of the implantable medical system, in particular a non-transvenous ICD, is spared. At the same time, a sufficiently precise estimation time is retrieved so that the implantable medical system can emit stimulation pulses either during the predicted T wave or outside the predicted T wave, depending on the desired result of the stimulation pulses.

The plurality of key sets may be understood to index the potential time of occurrence of the second event. Multiple potential times of occurrence of the second event may be indexed by a single key set. By doing so, the parameter allocation model may be kept to a desired (small) size. For example, there may be a number of 10 to 20 key sets, which index 30 to 40 potential times of occurrence the second event. Each of the key sets comprises a potential time of occurrence of the at least one first event. It may alternatively comprises a potential time of occurrence of two or three first events.

Generally, an acceleration of the heartbeat may lead to a shortening of a preceding diastolic interval on which the first event may be based which causes a shortening of the repolarization of the myocardium following the diastolic interval so that the time of occurrence of the second event is changed.

In an embodiment, the at least one first event represents a repolarization of the myocardium or a depolarization of the myocardium. Any first event detected in the cardiac electric signal may provide suitable (time) index for a potential time of occurrence of the second event. In particular, intervals between two subsequent ventricular contraction events, intervals between a ventricular contraction event and a subsequent T wave, and/or intervals between a T wave and a subsequent ventricular contraction event may be included as potential times of occurrence of at least one first event. Accordingly, the time of occurrence of the second event following one or more of these first events may be used as potential time of occurrence of the second event to which the time of occurrence of the one or more first events is mapped.

In an embodiment, the key set comprises a potential time of occurrence of a plurality of events wherein the potential time of occurrence of the plurality of first events (e.g., two or three times of occurrence) is mapped to the potential time of occurrence of the at least one second event. The parameter allocation model may, thus, be multidimensional in the sense that multiple key sets may be used to identify at least one potential time of occurrence of the second event. For example, a key set of the plurality of key sets may comprise a potential time of occurrence of a primary first event and a secondary first event. These two first events may be mapped to one or more potential times of occurrence of the second event.

The memory unit, according to the first aspect of the invention, stores a computer readable read-out program. When the computer readable read-out program is executed on the processor, it causes the processor to perform at least four steps.

In a first step, the cardiac electric signal comprising a plurality of events is received. The processor may analyze the cardiac electric signal to determine actual times of occurrence of the plurality of events. It may also analyze a type of event so that at least one first event may be identified.

In a second step, the actual time of occurrence is determined for at least one first event in the received cardiac signal. For example, the actual time of occurrence is determined as a distance in time between a current ventricular contraction event and a preceding ventricular contraction event.

In a third step, the key set in the list of key sets of the parameter allocation model is found that is closest to the actual time of occurrence of the at least one first event. Since each key set of the plurality of key sets comprises a potential time of occurrence of the at least one first event, there may be a key set that is closer to the actual time of occurrence than the other key sets (including identical). For example, each of the plurality of key sets may comprise a potential distance in time between the current ventricular contraction event and the preceding ventricular contraction event. The actual time of occurrence may be used by the processor to find the key set with the distance in time between the two contraction events that is closest to the actual time of occurrence. In such a case, each key set may only comprise the distance in time between the two contraction events.

The key set may also comprise potential times of occurrence of one or more additional first events. The actual times of occurrence of these one or more additional first events may be determined as well, in order to find the key set that is closest the determined actual times of occurrence of all first events.

In principle, the key set may be identical to the actual time of occurrence of the at least one first event. In such a case, the identical key set is the key set closest to the actual time of occurrence of the at least one first event.

In the fourth step, an estimation time is retrieved from the parameter allocation model. Herein, the estimation time represents an estimation of the time of occurrence of the second event. The retrieval of the estimation time is based on the at least one potential time of occurrence of the at least one second event to which the closest key set is mapped. In principle, the estimation time may be identical to the at least one potential time of occurrence of the at least one second event to which the closest key set is mapped. It may be advantageous, for example, to further include an analysis of the second closest key set in determining the estimation time. Retrieval of the estimation time may be finished before the at least one second event actually occurs. Executing computer readable read-out program may comprise configuring the processor so that the computer readable read-out program finishes operation before or at maximum when the at least one second event occurs.

In one embodiment, the parameter allocation model has the form of a look-up table. In such a case, the estimation time may be the at least one potential time of occurrence of the at least one second event to which the key set closest to the actual time of occurrence of the at least one first event is mapped. Hereby, the potential time of occurrence may be an aggregate of potential times of occurrence of several second events (e.g., an average).

In one embodiment, retrieval of the estimation time based on the potential time of occurrence of the second event to which the closest key set is mapped comprises interpolating the estimation time from the potential times of occurrence the second event to which the closest and second closest key sets are mapped or extrapolating the estimation time for the potential time of occurrence of the at least one second event. By using interpolation and/or extrapolation, a size of the parameter allocation model in terms of stored key sets and allocated potential times of occurrence may be kept small. For example, if an actual time of occurrence of at least one first event is between two key sets and rather far apart from both of them, an uncertainty in the potential time of occurrence of the second event may be minimized by interpolating between the potential times of occurrence of the second events to which the two key sets are mapped. If an actual time of occurrence of at least one first event is outside a range, a volume or higher dimensional limitation defined by the key sets of the parameter allocation model, one of the edge key sets may be closest to such an actual time of occurrence of at least one first event, but retrieval of a precise estimation time may be improved if the potential time of occurrence of the second event is extrapolated from the potential time of occurrence of the at least one second event to which the closest key set is mapped.

In one embodiment, potential times of occurrence of the at least one first event in the cardiac signal are formed by time intervals defining a rasterization into look-up classes. Such a rasterization may make finding the key set in the list of key sets of the parameter allocation model that is closest to the actual time of occurrence of the at least one first event easier because the look-up classes may form a continuous range of time values so that there are no gaps. If there are no gaps between the look-up classes, there is a higher chance that the actual time of occurrence of the at least one first event is identical to one of the look-up classes - saving further processing power for interpolating and, thus, battery life.

The time intervals defining a rasterization may be of equal length. In such a case, the rasterization may be equidistant. A length of the time intervals may as well vary. In an embodiment, the look-up classes are distinguished by width in time so that the rasterization is not equidistant. A shorter width in time may be provided at positions on the time axis where it is more likely that an actual time of occurrence of the at least one first event is located so that a more precise location of the at least one potential time of occurrence of the at least one second event to which the closest key set is mapped is possible.

In one embodiment, the look-up classes are anchored at an anchor point in time and extent 2% to 20% from the anchor point in both directions in time. Such an extension that depends on the value of the anchor point may compensate for measurement errors at small values of the actual time of occurrence of the at least one first event by providing broader look-up classes at such small values. Furthermore, by assigning each look-up class a predetermined extension in percent, the look-up classes may be adapted to have a similar or even identical density of potential times of occurrence of the at least one second event allocated to them.

In one embodiment, retrieving the estimation time comprises retrieving a representative value of several potential times of occurrence of the at least one second event to which the closest key set is mapped. The representative value may be an average or a median of the several potential times of occurrence of the at least one second event. After retrieving the representative value, the representative value may be updated using the actual time of occurrence of the at least one second event $C_n$. The update may include a weight coefficient a that assigns a weight to the representative value to be updated and to $C_n$. For example, the update of the representative value $\check{C}_{n-1}$ to an updated representative value $\check{C}_n$ may be performed by using the following equation:

$$\check{C}_n = \check{C}_{n-1} * a + (1-a) * C_n$$

The weight coefficient a may be a number between zero and one, preferably larger than 0.5 or larger than 0.9. The representative value may be calculated as part of the retrieval of the estimation time ("on-the-fly") from several potential times of occurrence of the at least one second event (e.g., as an average or median). The representative value may alternatively be readily provided by the memory unit.

The representative value may, in particular, be used in an embodiment in which the retrieval of the estimation time based on the potential time of occurrence of the second event to which the closest key set is mapped comprises interpolating the estimation time from the potential times of occurrence the second event to which the closest and second closest key sets are mapped or extrapolating the estimation time for the potential time of occurrence of the at least one second event. Herein, the potential time of occurrence of the second event may be any form of the representative value described above.

In an embodiment, the computer-readable read-out code causes the processor to generate at least one stimulation pulse emitted by the stimulation unit outside (i.e., prior to or after) the estimation time, e.g., an expected time of occurrence of a future T wave. Such a stimulation pulse emitted outside the future T wave is typically used for achieving a cardioversion of the patient's heart and for restoring a physiologic cardiac rhythm.

In an embodiment, the computer-readable read-out code causes the processor to emit at least one electric pulse by the stimulation unit or by a communication unit of the implantable medical system (e.g. a non-transvenous ICD) outside the estimation time. Such electric communication pulses should be typically not emitted during the occurrence of a T wave to avoid any risk of inducing ventricular fibrillation. Rather, by emitting electric communication pulses outside the estimation time, the risk of undesired cardiac effects due to the emitted electric pulses is significantly reduced.

Features and advantages have been described above in terms of time intervals. Alternatively, a heart rate may be used instead of the time interval without further deviation from the described features and advantages.

According to a second aspect of the invention, the object is achieved by an implantable medical system for stimulating a patient's heart, comprising a memory unit, a processor, a stimulation unit configured to stimulate the heart, and a detection unit configured to detect a cardiac electric signal. The memory unit stores a computer-readable record program that causes the processor to perform the following steps when executed on the processor:

- receiving a cardiac electric signal comprising a plurality of events,
- for at least one first event and at least one second event in the received cardiac electric signal, determining an actual time of occurrence,
- storing the actual time of occurrence of the at least one second event allocated to a key set corresponding to the actual time of occurrence of the at least one first event, so that the key set is mapped to the actual time of occurrence of the second event.

By repeating such a sequence of steps for several cardiac electric signals, a parameter application model may be gradually built that can be used by the computer readable read-out program according to the first aspect of the invention. Herein, the computer readable read-out program may be executed after execution (potentially multiple times) of the computer readable record program by the same implantable medical system.

For example, an actual time of occurrence may be determined for three first events and one second event. The actual time of occurrence of the three first events may form a key set that may be later used to estimate an estimation time according to the first aspect of the invention based on the actual time of occurrence of the at least one second event to which the key set is mapped.

In an embodiment, the memory unit comprises a ring buffer storage for each key set in which the actual time of occurrence of the at least one second event is stored. Each key set or a look-up class associated with the key set may form a memory pot which holds one or more times of occurrence of the at least one second event. Generally, older times of occurrence may be eliminated when younger times of occurrence advance in the memory pot to save memory capacity. A first-in-first-out (FIFO) memory may be a suitable way of implementing such a memory pot. The ring buffer or a shift register are possible implementations of such a memory pot.

In an embodiment, the memory unit is configured to store a representative value of several actual times of occurrence of at least one second event under one key set which representative value is updated when storing the actual time of occurrence of the at least one second event. Such an embodiment has already been described above in connection with the first aspect of the invention. It may realize a memory saving.

According to a third aspect of the invention, the object is achieved by a method for operating an implantable medical system. The method comprises two sets of steps. A first set and a second set. The first set of steps may be (repeatedly) performed before the second set of steps. First, a cardiac electric signal is received comprising a plurality of events.

According to the first set of steps:

a) For at least one first event and at least one second event in the received cardiac electric signal, an actual time of occurrence is determined and
b) the actual time of occurrence of the at least one second event allocated to a key set corresponding to the actual time of occurrence of the at least one first event is stored, so that the key set is mapped to the actual time of occurrence of the second event.

According to the second set of steps:

a) An actual time of occurrence is determined for at least one first event in the received cardiac electric signal.

b) Then a key set in a list of key sets of a parameter allocation model that is closest to the actual time of occurrence is found, and

c) an estimation time from the parameter allocation model is retrieved, which represents an estimation of a time of occurrence of a second event, based on an at least one potential time of occurrence of the at least one second event to which the closest key set is mapped.

All embodiments of the arrangement can be combined in any desired way and can be transferred either individually or in any arbitrary combination to the described method. Likewise, all embodiments of the described method can be combined in any desired way and can be transferred either individually or in any arbitrary combination to the described arrangement.

Further details of aspects of the present invention will be explained in the following making reference to exemplary embodiments and accompanying Figures. In the Figures:

Figure 1        shows a diagram of the action potential duration over various diastolic intervals;

Figure 2A       shows a schematic depiction of the application of a T wave shock in a transvenous ICD known from prior art;

Figure 2B       shows the application of a cardioversion in a common transvenous ICD known from prior art;

Figure 3        shows an example of an implantable medical system;

Figure 4A       shows a parameter allocation model with several key sets and a cardiac electric signal used in observation mode;

Figure 4B       shows a diagram of the information contained in the parameter allocation model of Figure 4A;

Figure 5        shows a parameter allocation model with several key sets and a cardiac electric signal used in read-out mode;

Figure 6        shows a parameter allocation model with rasterization (equidistant);

Figure 7        shows a parameter allocation model with rasterization (not equidistant);

Figure 8        shows a two-dimensional parameter allocation model;

Figure 9        shows various possibilities of signal identification;

Figure 10A      shows an embodiment of a programming device;

Figure 10B      shows examples of pattern tracking in a cardiac electric signal;

Figure 11       shows an example of a two-step signal analysis;

Figure 12       schematically shows cooperation between a programming device and an ICD;

Figure 13       schematically shows cooperation between a programming device and an ICD including a surface ECG; and

Figure 14       shows the implantable medical system together with a schematic torso.

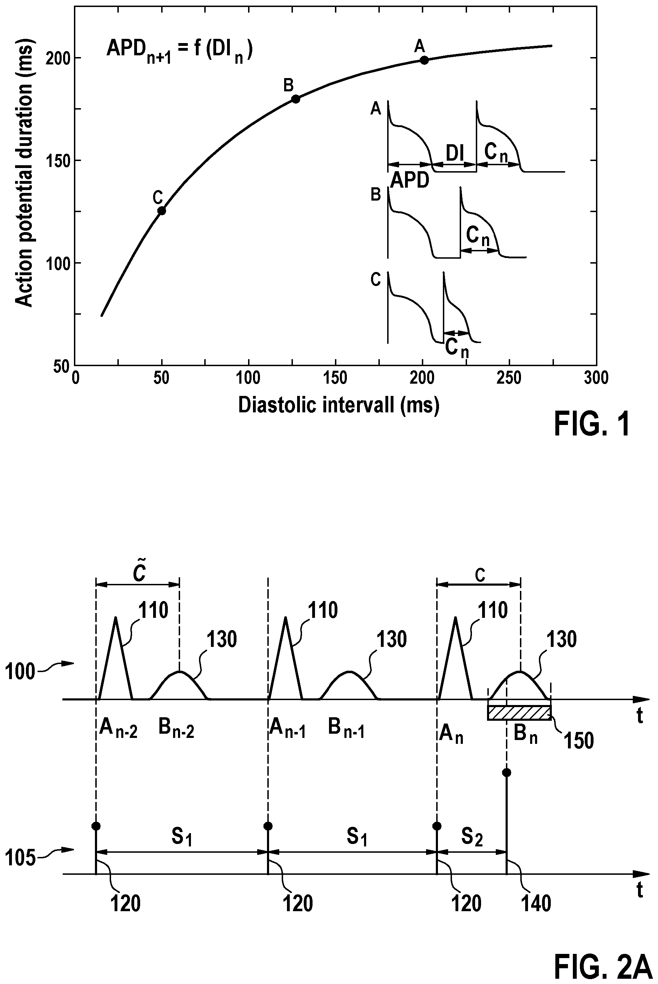

Figure 1 shows a chart of the action potential duration over various diastolic intervals according to Jordan and Christini, "Therapies for ventricular cardiac arrhythmias" Critical reviews in biomedical engineering 33 6 (2005): 557-604. It can be seen that the action potential duration is a function of the preceding diastolic interval. If the preceding diastolic interval gets shorter, the action potential duration also decreases in length. A decrease in the diastolic interval corresponds to an acceleration of the heart rhythm. Thus, it can be concluded that the action potential duration varies in dependence of the heart rhythm. The variation of the action potential duration makes it hard to estimate precisely. Precise estimation, however, is required, for example, for initiation of T wave shocks. Patient's well-being benefits from being able to apply stimulation pulse at a safe time. Precise estimation of a potential time of occurrence of a T wave may also support implantable medical device to filter out noise or undesired peaks when planning a T wave stimulation.

Figure 2A exemplarily shows the application of a T wave shock with an implantable medical system in form of a common transvenous ICD 200 according to prior art. On a measuring channel 100, electrocardiogram signals representing cardiac activity are sensed. "A" events 110 ($A_n$, $A_{n-1}$, $A_{n-2}$; representing cardiac depolarization which can typically best be observed by an R wave of the QRS complex) are typically enforced by a synthetic cardiac rhythm due to regularly applied stimulation pulses 120. These stimulation pulses 120 are applied in a temporal distance S1. These stimulation pulses 120 are emitted on an output channel 105 of the ICD 200 system.

The occurrence of a "B" event 130 (corresponding to a T wave in the electrocardiogram) is typically manually determined. For this purpose, a value $\tilde{C}$ is measured that corresponds to the distance between the "B" event 130 and the preceding "A" event 110. This value $\tilde{C}$ is set as a fixed value C in the transvenous ICD 200 system. The fixed value C can be, e.g., an average value of a plurality of $\tilde{C}$ values.

The time point of emitting a shock pulse 140 is calculated by $S_2 = \kappa * C$. Then, the shock pulse 140 is emitted within the vulnerable window 150 of the ventricles to be stimulated. Consequently, a T wave shock resulting in ventricular fibrillation is applied within the relevant time window.

The enforced stimulation ensures a relatively constant value for C. Such enforced stimulation is, however, an additional stress for the patient in case of non-transvenous ICD 200 systems and is therefore to be avoided.

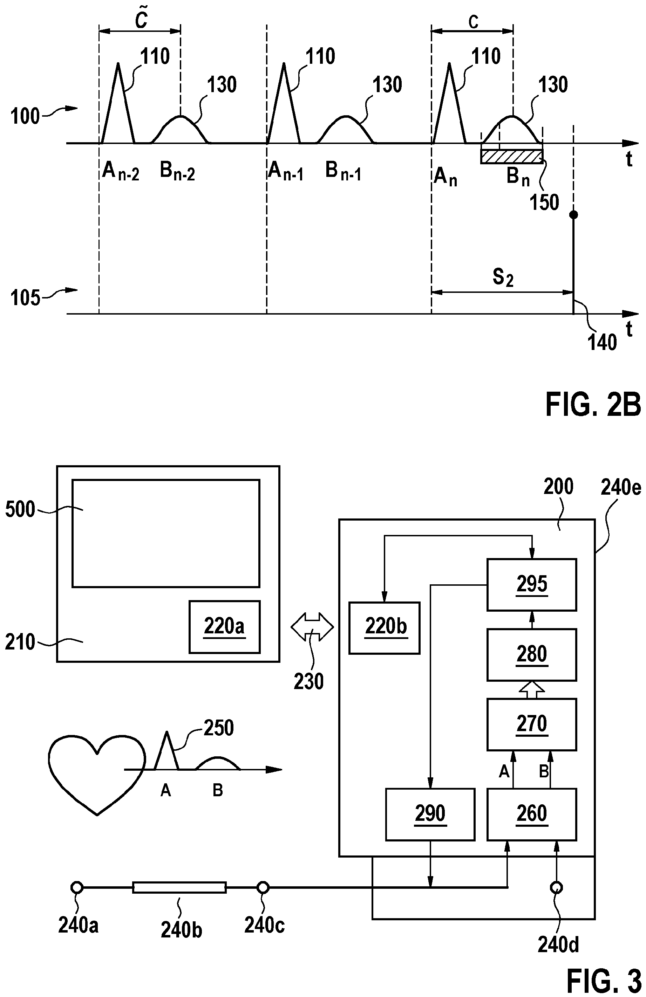

Figure 2B exemplarily shows a cardioversion process also known from prior art. In this and in all following Figures, similar elements will be denoted with the same numeral reference.

The different "A" events 110 are automatically detected by the ICD 200. The distance C is again determined by manually measuring the distance $\tilde{C}$ between the "B" event (T wave) 130 and the directly preceding "A" event 110 (R wave of a QRS complex). The time point of emitting a shock pulse 140 is calculated as $S_2 = \kappa * C$ so that the shock pulse 140 is emitted outside the vulnerable window 150 of the ventricles. Then, the shock pulse 140 results in a cardioversion, not in ventricular fibrillation like in case of a T wave shock as illustrated in Figure 2A.

A problem occurs if the intrinsic cardiac rhythm significantly varies since this results in a variation of the correct value of C. Furthermore, the effects of cardiac restitution are to be considered. An acceleration of the cardiac rhythm results in a shortening of a preceding diastolic interval and thus in a shortening of the repolarization duration as shown in Figure 1. Due to this shortening, the calculated value $S_2$ for the shock pulse 140 to be applied outside the vulnerable window 150 of the ventricles can erroneously fall within the vulnerable phase.

Likewise, the shock pulse 140 can also be erroneously applied outside the vulnerable window 150 even though it was intended to be applied within the vulnerable window 150 (like in case of Figure 2A).

This illustrates the need for an exact determination of the occurrence of a future T wave, i.e. of the distance C.

Figure 3 shows an exemplary implantable medical system comprising a non-transvenous implantable cardioverter defibrillator device (ICD 200) and a programming device 210 operatively coupled to the non-transvenous ICD 200. Specifically, the programming device 210 and the ICD 200 comprise communication units configured to communicate wirelessly in two directions 230. The programming device 210 is provided to configure the ICD 200 via a user interface 500.

The ICD 200 comprises a processor formed by a predictor 280, a delay device 295 and a generator unit 290. The processor receives, during operation, a cardiac electric signal 250 that is provided by a sensing unit 260 operatively coupled to at least one first electrode pole 240a-e (in this case five electrode poles including a housing 240e of the ICD 200) configured to sense the cardiac electric signal 250. The sensing unit 260 is configured to detect a plurality of first events A, B within the cardiac electric signal 250. It is operatively coupled to a memory unit 270 provided to store an actual time of occurrence of first and second events A,B. The actual time of occurrence of the first and second events A, B may be determined by the sensing unit 260 or a separately provided time determination unit. The predictor 280 is configured to retrieve an estimation time which represents an estimation of the time of occurrence of a second event B, based on an actual time of occurrence of the first and/or second events A, B.

The estimation time is used by the delay device 295 to delay application of electric pulses via at least two electrode poles 240a-d to the patient's heart.

For example, the predictor 280 determines a plurality of "AA" intervals between subsequent "A" events. Alternatively or additionally, the predictor 280 identifies a plurality of "C" intervals between an "A" event and a subsequent "B" event. Alternatively or additionally, the predictor 280 identifies a plurality of "D" intervals between a "B" event and a subsequent "A" event. Accordingly, the delay unit delays an application of an electric pulse through the generator unit 290 by a time of an "AA", "C" or "D" interval.

Some of the at least one first electrode poles 240a-d may be used as shock electrodes and vice versa. One or several of the electrode poles 240a-d may be formed as rings and/or spirals. One or several of the electrode poles 240a-d may be disposed on the housing 240e of the ICD 200 (raised, level or recessed).

The sensing unit may comprise one or more of the following units for detecting a plurality of events within a cardiac electric signal 250: an optional prefiltering unit, a triggering unit with programmable and/or adaptive thresholding, a peak detection unit, a signal deviation detection unit, in particular designed for the detection in time windows of programmable width, and a heart rate determination unit configured to determine a heart rate using an algorithm, for example based on Pan-Tomkins algorithm.

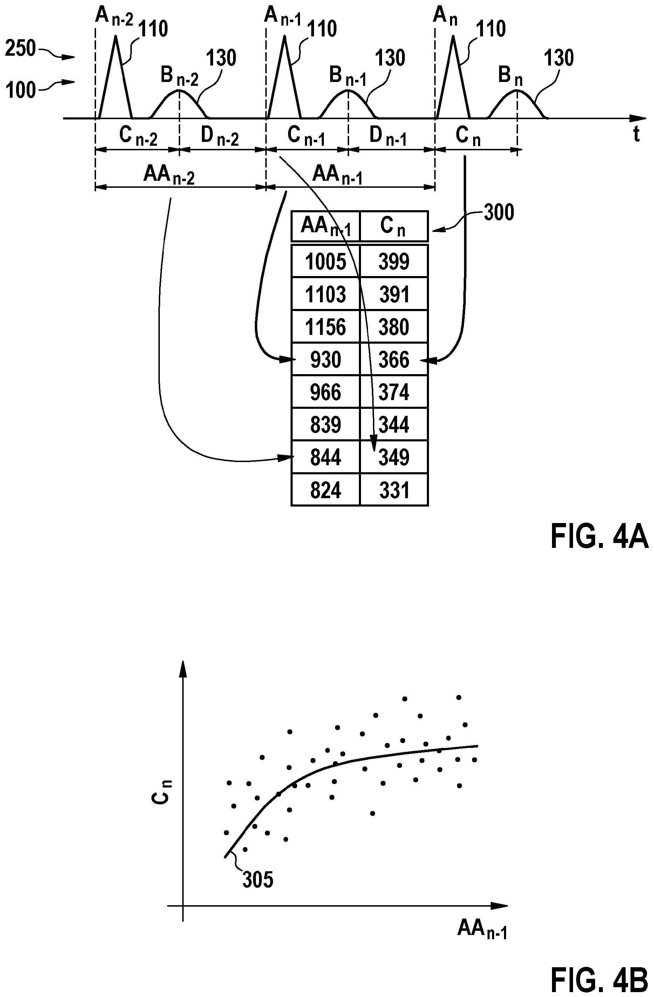

Figure 4A illustrates the times of occurrence of first and second events A, B in the form of metrics or intervals in a cardiac electric signal 250 (measured in a measuring channel 100). These may be determined by the sensing unit 260 or a separately provided time determining unit as described above (cf. Figure 3). Generally, the cardiac electric signal 250 may be from the same or different cardiac signal derivatives, which are understood as potential differences of electrode pole 240a-d pairs. In particular, they can also originate from intra- and extracorporeal sources.

"AA" intervals are measured between two subsequent "A" events 110. "C" intervals are measured between a preceding "A" event 110 and a subsequent "B" event 130. Finally, "D" intervals are measured between a preceding "B" event 130 and a subsequent "A" event 110. Apparently, there is an interdependence between "AA" intervals, "C" intervals and "D" intervals. However, the individual intervals cannot necessarily be calculated from each other since the cardiac rhythm is subject to physiologic (and sometimes to non-physiologic) variations and the determined values may originate from different derivatives. Therefore, "AA" intervals, "C" intervals, and/or "D" intervals generally contain different timing information on the cardiac rhythm and can be used together or alternatively for determining an estimation time which represents an estimation of the time of occurrence of the next future "B" event 130.

For the determining of the estimation time, actual times of occurrence of "AA" intervals and "C" intervals are stored in a look-up table. Hereby, actual times of occurrence in the form of "AA" intervals are allocated to "C" intervals so that each preceding "AA" interval forms a key set for a following "C" interval. In this way, key sets corresponding to an actual time of occurrence of a first event A (A event 110) in a first column 310 of the look-up table are mapped to the actual time of occurrence of a second event B (B event 130) in a second column 320 of the look-up table. In principle, one or more of "AA" intervals, "C" intervals, and/or "D" intervals such as $AA_{n-1}$, $C_{n-1}$ and/or $D_{n-1}$ may be stored as key sets mapped to the actual time of occurrence of the second event $C_n$.

Storing of actual times of occurrence may be carried out in an observation mode of operation of the implantable medical system. During the observation mode several times of occurrence are stored in the look-up table providing potential times of occurrence that may be used to estimate a time of occurrence of future events.

Figure 4B illustrates the times of occurrence stored in the look-up table shown in Figure 4A in a graph showing the times of occurrence of $C_n$ plotted over times of occurrence of events $AA_{n-1}$ preceding $C_n$. A trendline 305 approximately shows a trend in the data points that increases strongly at smaller "AA" intervals (i.e., a relatively strong increase of $C_n$ at small "AA" intervals) and less strongly at larger "AA" intervals (i.e., a relatively weak increase of $C_n$ at large "AA" intervals).

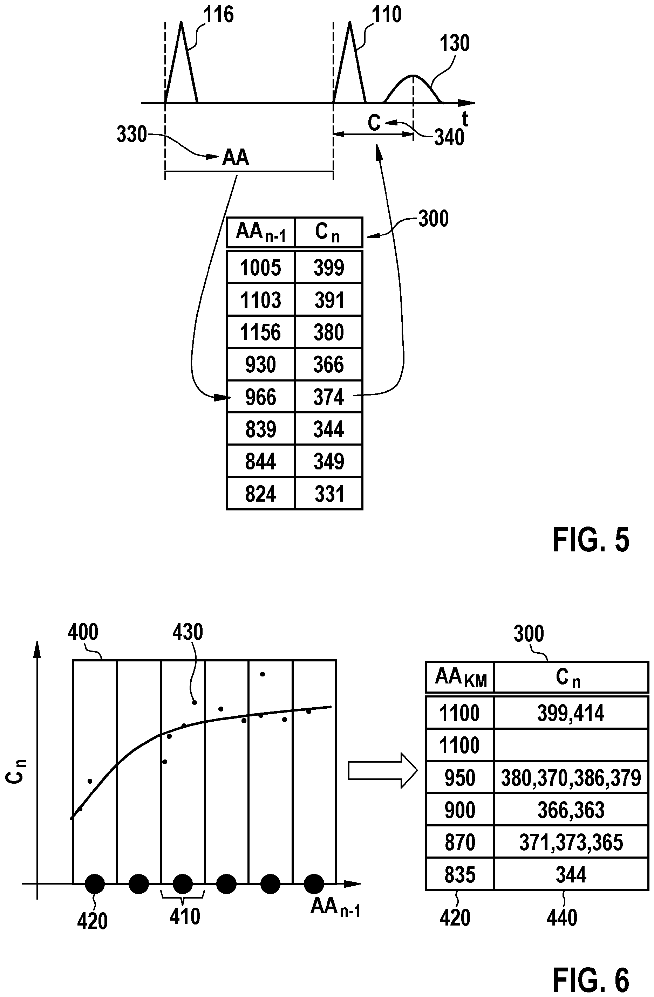

Figure 5 illustrates a read-out mode of operation. The determined actual time of occurrence of at least one first event 330 (in this case an "AA" interval) is searched in the list of key sets of the look-up table 300. The key set closest (in this case identical) to the determined actual time of occurrence is found and an estimation time which represents an estimation of the time of occurrence of a second event 340 (the "C" interval) is retrieved.

Figure 6 shows a rasterization of potential times of occurrence of a first event. The entire time span, that is covered by determined times of occurrence of the first event (in this case time interval $AA_{n-1}$) is rasterized into time intervals of a predetermined length (in this case equal length), thereby forming look-up classes 400 of potential times of occurrence of the first event A with a class width 410 corresponding to the length of the time intervals used for the rasterization. Each class 400 is centered around an anchor point (illustrated by a large dot). Potential times of occurrence of one or more second events $C_n$ (data points 430 and second column 440) are allocated to each class 400 (first column with class centers 420). A dedicated memory unit 270 comprising a ring buffer storage for each key set may be provided in which the potential times of occurrence of the at least one second event $C_n$ are stored. The value of the class center 420 is used to index the class 400 for the purpose of finding it as a key set in the look-up table.

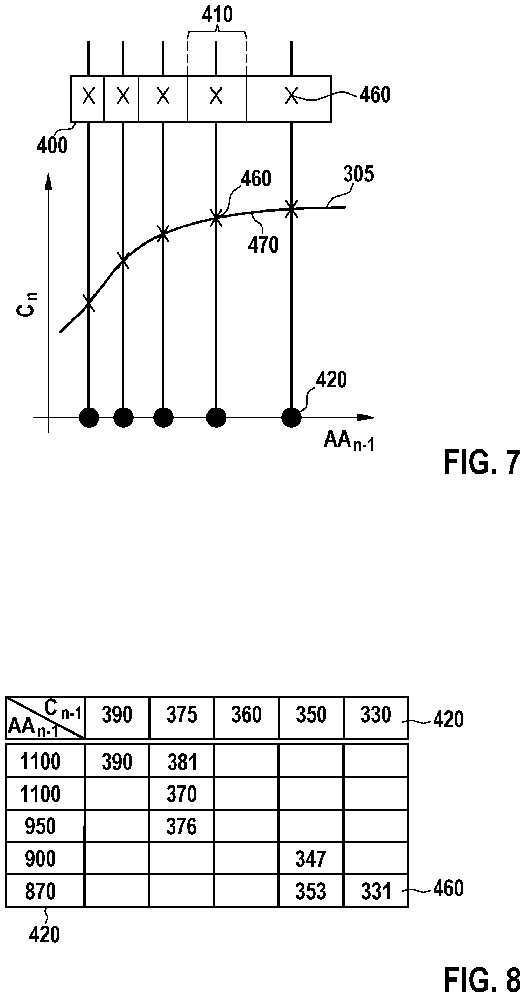

Figure 7 shows another example of a rasterization of potential times of occurrence of a first event. The time span, that is covered by determined times of occurrence of the first event (in this case time interval $AA_{n-1}$) is rasterized into time intervals of a predetermined length, thereby forming look-up classes 400 of potential times of occurrence of the first event with a class width 410 corresponding to the length of the time intervals used for the rasterization. Here, the time intervals have different predetermined lengths. The look-up classes 400 are, thus, distinguished by width in time so that the rasterization is not equidistant. The width in time is shorter where the trendline 305 is steeper so that a higher resolution may be achieved in case of small $AA_{n-1}$. At higher $AA_{n-1}$, the width in time may larger.

Furthermore, each class 400 contains only a single potential time of occurrence of a second event B. In the operation mode, actual times of occurrence of second events B may be added to a class 400 by updating the stored potential time of occurrence of second event B. For this purpose, the single potential time of occurrence of the second event 460 may be a representative value, for example an average or a median of previously determined times of occurrence of the second event B. In the read-out mode, the estimation time may be retrieved as the representative value of the respective key set that is closest to the queried actual time of occurrence of the first event. Alternatively, the estimation time may be retrieved as an interpolated or extrapolated value from two representative values of the classes 400 closest to the actual time of occurrence of the first event.

Figure 8 shows a look-up table which has key sets comprising a potential time of occurrence of two first events ("$AA_{n-1}$" and "$C_{n-1}$" intervals). The potential time of occurrence of the two first events is mapped to the potential time of occurrence of one second event 460. Thus, the look-up table is two-dimensional. Furthermore, the two potential times of occurrence of the first event are rasterized, thereby forming two-dimensions of look-up classes 400. As can be seen from the values of the class centers 420 of the respective rasterized potential times of occurrence of the first events, the look-up classes 400 are distinguished by width in time so that the rasterization is not equidistant.

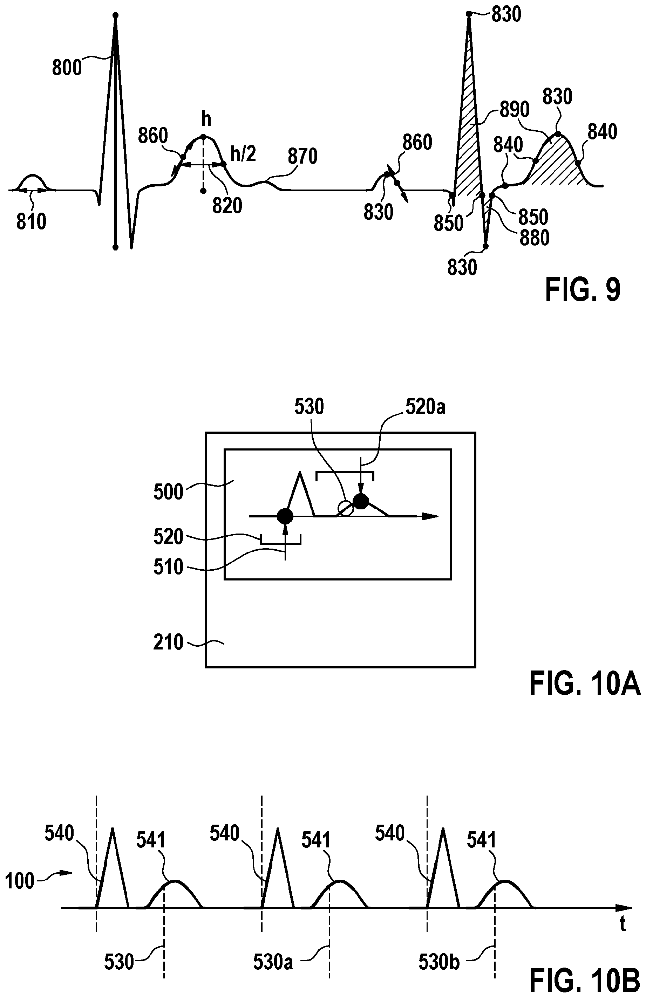

Figure 9 illustrates various possibilities of signal identification during the processing of the electrocardiogram signals. A signal height 800, a signal width 810, and a full width at half maximum 820 are particular appropriate measures for identifying signals in the electrocardiogram. In addition, the amount of extrema 830, inflection points 840, and/or zero crossings 850 are appropriate measures. Likewise, a slope 860, a curvature 870 as well as change of signs 880 or areas 890 bearing a positive sign are appropriate measures for signal identification.

Figure 10A shows an embodiment of a programming device 210. This programming device 210 bears an interactive display 500 that enables the determination of patterns for cardiac depolarization and/or cardiac repolarization. To give an example, these patterns can be marked with a cursor, a mouse or a touchscreen and stored as pattern marker 510. Signal sections in a window 520 around the pattern marker 510 are then used as target pattern for pattern tracking. It is also possible to additionally mark a characteristic point or section within the pattern marker 510 that may have a fixed relative position to the pattern to be identified. In case of a T wave shock, the target pattern for the T wave can be anchored at a central point 520a within the target pattern. Nonetheless, it is possible to emit the T wave shock with a relative offset to this central point 520a. In the embodiment of Figure 10A, a T wave shock emission marker 530 marks the time point at which the T wave shock is to be applied.

Figure 10B shows further details of the pattern tracking applied by the programming device 210 of Figure 10A (and a non-transvenous ICD 200 coupled with this programming device 210, but not illustrated in Figures 10A and 10B).

A first target pattern 540 and a second target pattern 541 that have been determined once, can be easily identified in subsequent cardiac events by evaluating only discrete signal portions 540a, 541a, 540b, and 541b in those subsequent electrocardiogram signals. In the embodiment shown in Figure 10B, this is done with a correlation waveform analysis (CWA). For such analysis, a correlation coefficient is calculated between the first target pattern 540 and the electrocardiogram signals in an evaluation window covering subsequent cardiac signals. If this correlation coefficient is higher than a predetermined threshold, the respective signal is identified as a kind of signal to which the first target pattern 540 has been assigned. Preferably, the pattern-tracking algorithm is performed in the non-transvenous ICD 200. Based on the additional signals identified within the electrocardiogram, subsequent T wave shock emission markers 530a and 530b are determined in the same way as the first T wave shock emission marker 530 has been determined.

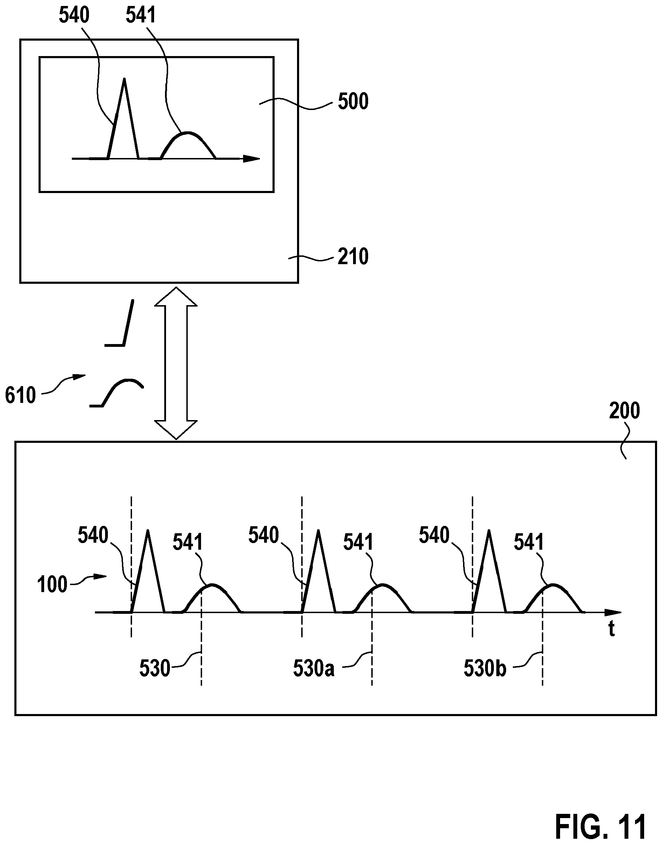

Figure 11 illustrates an embodiment in which a two-step signal analysis is performed. In a programming device 210 having an interactive display 500 for pattern identification, computing-intensive operations for identifying a first target pattern 540 and a second target pattern 541 are performed. The first target pattern 540 and the second target pattern 541 are then transferred to a non-transvenous ICD 200 operatively coupled with the programming device 210. Here, less computing-intensive operations are performed to identify patterns within the electrocardiogram signal corresponding to the first target pattern 540 and/or the second target pattern 541. Reference is made to the explanations given with respect to Figure 10B. These less computing-intensive operations are, e.g., a correlation with deconvolution to identify the first target pattern 540 and/or the second target pattern 541 in signals representing subsequent cardiac events in real time. Due to a bidirectional communication 610 between the programming device 210 and the non-transvenous ICD 200, it is possible to transfer the sites of the electrocardiogram in which the first target pattern 540 and the second target pattern 541 have been identified to the programming device 210. The programming device 210 can then double-check the identified patterns and/or visualize them on the interactive display 500.

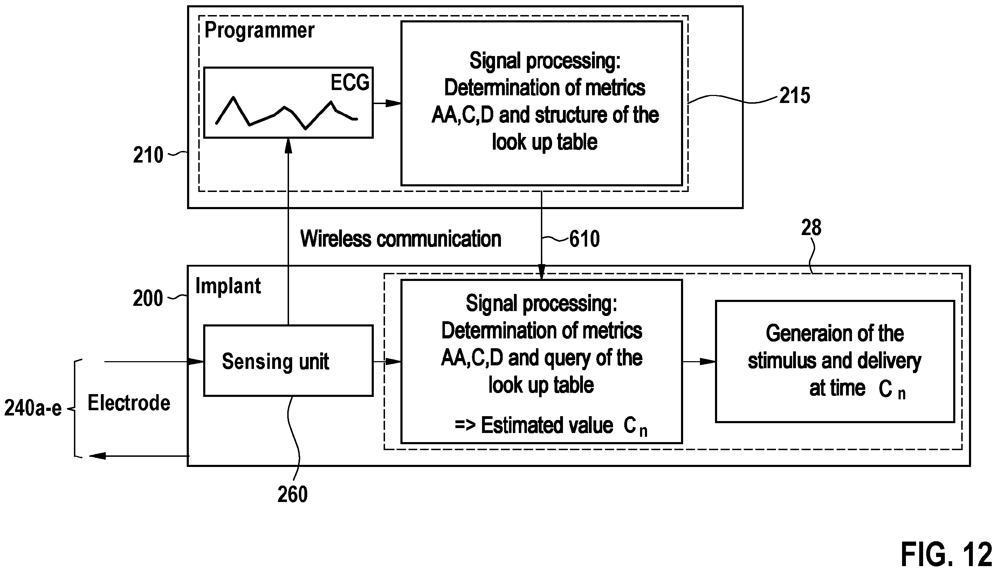

Figure 12 illustrates an implantable medical system for stimulating a patient's heart. In general, the patient may be a human or an animal. The implantable medical system comprises an ICD 200 and a programming device 210. The ICD 200 is equipped with a detection unit in the form of a sensing unit 260 comprising several electrode poles 240a-d. The cardiac electric signal 250 is detected through this detection unit. The detected cardiac electric signal 250 may be evaluated in the ICD 200 or transmitted to the programming device 210 for evaluation by a signal processing unit 215 - leaving the evaluation to the programming device 210 may save battery resources at the ICD 200. For transmission, the ICD 200 is operatively connected via a bidirectional communication 610 to the programming device 210.

Evaluation of the detected cardiac electric signal comprises detecting a plurality of events within the cardiac electric signal and determining an actual time of occurrence for at least one first event A. The actual time of occurrence may be one or more of an "AA" interval, a "C" interval or a "D" interval. Then, a parameter allocation model comprising a plurality of key sets, each of which comprises a potential time of occurrence of at least one first event A in the cardiac electric signal and each of which is mapped to at least one potential time of occurrence of the second event in the cardiac electric signal, is used when finding the key set that is closest to the actual time of occurrence. Based on this query, an estimation time $C_n$ is retrieved from the parameter allocation model, which represents an estimation of the time of occurrence of the second event B based on the at least one potential time of occurrence of the at least one second event B to which the closest key set is mapped. The described evaluation of the detected cardiac signal is performed in read-out mode by (e.g. a processor 28 of) the ICD 200. Alternatively, the evaluation may be carried out by the programming device 210 (e.g., at its signal processing unit 215).

Finally, at least one stimulation pulse or at least one electric pulse are generated and emitted (e.g., by the processor 28) at the estimation time.

In an observation mode, evaluation of the detected cardiac signal may comprise determining an actual time of occurrence for at least one first event A and at least one second event B in the received cardiac electric signal 250. After detection, the actual time of occurrence of the at least one second event B is stored allocated to a key set corresponding to the actual time of occurrence of the at least one first event A so that the key set is mapped to the actual time of occurrence of the second event B. In this way a parameter allocation model may be gradually built. Such an evaluation may preferably be performed by the programming device 210. The programming device 210 may transmit the parameter allocation model (once it is built) to the ICD 200 for further evaluation in the read-out mode.

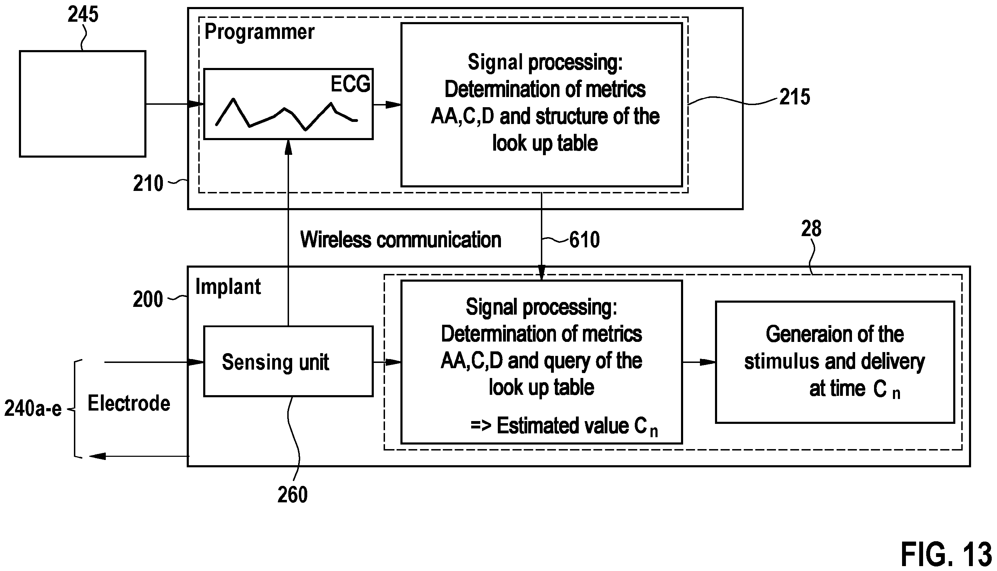

Figure 13 illustrates the implantable medical system of Figure 12 including additional surface electrodes 245 operatively connected to the programming device 210 providing the programming device 210 with a surface ECG. The signal processing unit 215 of the programming device 210 receives the surface ECG and the cardiac electric signal detected by the ICD 200. The surface ECG is evaluated concurrently with the cardiac electric signal so that two different sources may be used to determine metrics and intervals advantageously increasing preciseness of the estimation time. The estimation time may be transmitted to the ICD 200 and passed on to the generator unit 290 so that an electric pulse can be emitted at the estimation time. Alternatively, to emitting the electric pulse at the estimation time, the electric pulse may be emitted within an offset to the estimation time that may be predetermined.

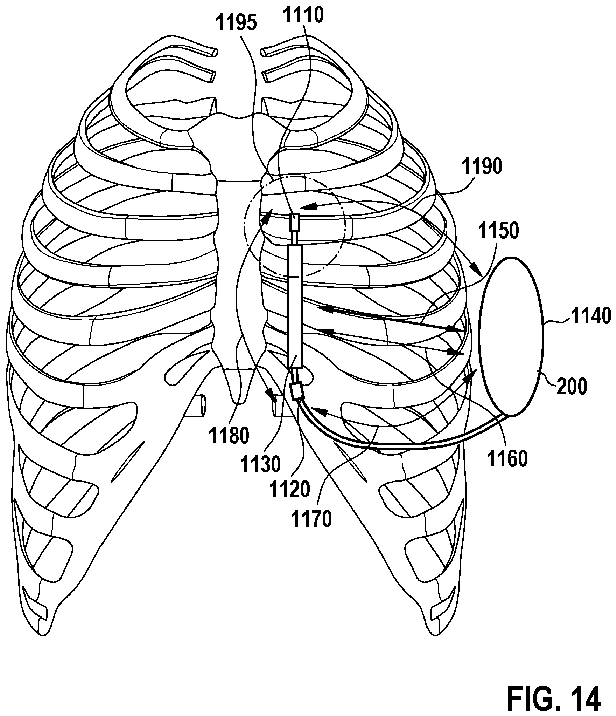

Figure 14 shows the implantable medical system with respect to a schematic human torso. The implantable medical system comprises four electrodes that are used as sensing poles 110, 1120, 1130, 1140 spanning four sensing vectors 1160, 1170, 1180, 1190 between them. Two of these electrodes are additionally used as shock poles 1130, 1140 spanning a shock vector 1150 between them. The electrode poles 240a-d may be implanted within a tolerance of 10 cm or 5 cm.

## Claims

1. Implantable medical system for stimulating a patient's heart, comprising a memory unit (270), a processor (28), a stimulation unit (290) configured to stimulate the heart, and a detection unit (260) configured to detect a cardiac electric signal (250), **characterized in**

   **that** the memory unit (270) stores a parameter allocation model which comprises a plurality of key sets (310, 420), each of which comprises a potential time of occurrence of at least one first event (110) in the cardiac electric signal (250) and each of which is mapped to at least one potential time of occurrence of a second event (130) in the cardiac electric signal (250) representing a repolarization of the myocardium, wherein
   the memory unit (270) further stores a computer-readable read-out program that causes the processor (28) to perform the following steps when executed on the processor (28):

      - receiving a cardiac electric signal (250) comprising a plurality of events (110),
      - for at least one first event (110) in the received cardiac electric signal (250), determining an actual time of occurrence,
      - finding the key set in the list of key sets of the parameter allocation model that is closest to the actual time of occurrence, and
      - retrieving an estimation time from the parameter allocation model, which represents an estimation of the time of occurrence of the second event (130), based on the at least one potential time of occurrence of the at least one second event (130) to which the closest key set is mapped.

2. Implantable medical system according to claim 1, **characterized in that** the at least one first event (110) represents a repolarization of the myocardium or a depolarization of the myocardium.

3. Implantable medical system according to one of claims 1 or 2, **characterized in that** the key set comprises a potential time of occurrence of a plurality of first events (110) wherein the potential time of occurrence of the plurality of first events (110) is mapped to the potential time of occurrence of the at least one second event (130).

4. Implantable medical system according to one of claims 1 to 3, **characterized in that** the parameter allocation model has the form of a look-up table (300).

5. Implantable medical system according to one of the preceding claims, **characterized in that** retrieval of the estimation time based on the potential time of occurrence of the second event (130) to which the closest key set is mapped comprises interpolating the estimation time from the potential times of occurrence of the second event (130) to which the closest and second closest key sets are mapped or extrapolating the estimation time from the potential time of occurrence of the at least one second event (130).

6. Implantable medical system according to one of the preceding claims, **characterized in that** potential times of occurrence of the at least one first event (110) in the cardiac electric signal (250) are formed by time intervals defining a rasterization into look-up classes (400).

7. Implantable medical system according to claim 6, **characterized in that** look-up classes (400) are distinguished by width in time so that the rasterization is not equidistant.

8. Implantable medical system according to one of claims 6 or 7, **characterized in that** the look-up classes (400) are anchored at an anchor point in time and extend 2 % to 20 % from the anchor point in both directions in time.

9. Implantable medical system according to one of the preceding claims, **characterized in that** retrieving the estimation time comprises retrieving a representative value of several potential times of occurrence of the at least one second event (130) to which the closest key set is mapped.

10. Implantable medical system according to one of the preceding claims, **characterized in that** the computer-readable read-out code causes the processor (28) to generate at least one stimulation pulse emitted by the stimulation unit (290) during or outside the estimation time.

11. Implantable medical system according to one of the preceding claims, **characterized in that** the computer-readable read-out code causes the processor (28) to emit at least one electric pulse by the stimulation unit (290) or a communication unit of the implantable medical system outside the estimation time.

12. Implantable medical system for stimulating a patient's heart, comprising a memory unit (270), a processor (28), a stimulation unit (290) configured to stimulate the heart, and a detection unit (260) configured to detect a cardiac electric signal (250), **characterized in that** the memory unit (270) stores a computer-readable record program that causes the processor (28) to perform the following steps when executed on the processor (28):

- receiving a cardiac electric signal (250) comprising a plurality of events,
- for at least one first event (110) and at least one second event (130) in the received cardiac electric signal (250), determining an actual time of occurrence,
- storing the actual time of occurrence of the at least one second event (130) allocated to a key set corresponding to the actual time of occurrence of the at least one first event (110), so that the key set is mapped to the actual time of occurrence of the second event (130).

13. Implantable medical system according to claim 12, **characterized in that** the memory unit (270) comprises a ring buffer storage for each key set in which the actual time of occurrence of the at least one second event (130) is stored.

14. Implantable medical system according to claim 12, **characterized in that** the memory unit (270) is configured to store a representative value of several actual times of occurrence of at least one second event (130) under one key set which representative value is updated when storing the actual time of occurrence of the at least one second event (130).

15. Method for operating an implantable medical system, in particular according to any of the preceding claims, **characterized by** receiving a cardiac electric signal (250) comprising a plurality of events, and one or both of the following sets of steps:

a) for at least one first event (110) and at least one second event (130) in the received cardiac electric signal (250), determining an actual time of occurrence and
b) storing the actual time of occurrence of the at least one second event (130) allocated to a key set corresponding to the actual time of occurrence of the at least one first event (110), so that the key set is mapped to the actual time of occurrence of the second event (130); or
a) for at least one first event (110) in the received cardiac electric signal (250), determining an actual time of occurrence,
b) finding a key set in a list of key sets of a parameter allocation model that is closest to the actual time of occurrence, and
c) retrieving an estimation time from the parameter allocation model, which represents an estimation of a time of occurrence of a second event (130), based on an at least one potential time of occurrence of the at least one second event (130) to which the closest key set is mapped.

$$APD_{n+1} = f(DI_n)$$

FIG. 1

FIG. 2A

FIG. 2B

FIG. 3

FIG. 4A

FIG. 4B

FIG. 5

| $AA_{n-1}$ | $C_n$ |
|---|---|
| 1005 | 399 |
| 1103 | 391 |
| 1156 | 380 |
| 930 | 366 |
| 966 | 374 |
| 839 | 344 |
| 844 | 349 |
| 824 | 331 |

| $AA_{KM}$ | $C_n$ |
|---|---|
| 1100 | 399,414 |
| 1100 | |
| 950 | 380,370,386,379 |
| 900 | 366,363 |
| 870 | 371,373,365 |
| 835 | 344 |

FIG. 6

410

X X X X X — 460

400

C_n

460    305

470

420

AA_{n-1}

**FIG. 7**

| AA_{n-1} \ C_{n-1} | 390 | 375 | 360 | 350 | 330 | |
|---|---|---|---|---|---|---|
| 1100 | 390 | 381 | | | | |
| 1100 | | 370 | | | | |
| 950 | | 376 | | | | |
| 900 | | | | 347 | | |
| 870 | | | | 353 | 331 | 460 |

420                                           420

**FIG. 8**

FIG. 9

FIG. 10A

FIG. 10B

FIG. 11

FIG. 12

FIG. 13

FIG. 14

# EP 4 736 936 A1

**EUROPEAN SEARCH REPORT**

Application Number

EP 24 21 0873

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2003/130705 A1 (MORRIS MILTON M [US]) 10 July 2003 (2003-07-10) | 12,13,15 | INV. A61N1/365 |
| A | * the whole document * | 1-11,14 | A61B5/349 A61N1/39 |
| A | US 2023/107061 A1 (GREENHUT SAUL E [US] ET AL) 6 April 2023 (2023-04-06) * abstract * * paragraph [0135] - paragraph [0401] * * figures 1-23 * | 1-15 | |
| A | US 2012/046563 A1 (ALLAVATAM VENUGOPAL [US] ET AL) 23 February 2012 (2012-02-23) * the whole document * | 1-15 | |

TECHNICAL FIELDS SEARCHED (IPC)

A61N
A61B

The present search report has been drawn up for all claims

| Place of search | Date of completion of the search | Examiner |
|---|---|---|
| Munich | 1 April 2025 | Artikis, T |

EPO FORM 1503 03.82 (P04C01)

## ANNEX TO THE EUROPEAN SEARCH REPORT
## ON EUROPEAN PATENT APPLICATION NO.

EP 24 21 0873

This annex lists the patent family members relating to the patent documents cited in the above-mentioned European search report.
The members are as contained in the European Patent Office EDP file on
The European Patent Office is in no way liable for these particulars which are merely given for the purpose of information.

01-04-2025

| Patent document cited in search report | Publication date | Patent family member(s) | | Publication date |
|---|---|---|---|---|
| US 2003130705 A1 | 10-07-2003 | AU | 7120600 A | 10-04-2001 |
| | | US | 6480741 B1 | 12-11-2002 |
| | | US | 2003130705 A1 | 10-07-2003 |
| | | WO | 0117609 A1 | 15-03-2001 |
| US 2023107061 A1 | 06-04-2023 | NONE | | |
| US 2012046563 A1 | 23-02-2012 | US | 2012046563 A1 | 23-02-2012 |
| | | US | 2018220916 A1 | 09-08-2018 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **BAROLD, H. S.** ; **WHARTON, J. M.** Ventricular fibrillation resulting from synchronized internal atrial defibrillation in a patient with ventricular preexcitation. *Journal of Cardiovascular Electrophysiology*, 1997, vol. 8 (4), 436-440

- **WYLIE JR, J. V** ; **ESSEBAG, V.** ; **REYNOLDS, M. R** ; **JOSEPHSON, M. E**. Inducibility of Atrial Fibrillation with a Synchronized External Low Energy Shock Post-Pulmonary Vein Isolation Predicts Recurrent Atrial Fibrillation.. *Journal of Cardiovascular Electrophysiology*, 2009, vol. 20 (1), 29-36
- **JORDAN** ; **CHRISTINI**. Therapies for ventricular cardiac arrhythmias. *Critical reviews in biomedical engineering*, 2005, vol. 33 (6), 557-604